# EUROPEAN PATENT APPLICATION

(11) **EP 4 548 938 A1**
(43) Date of publication of application: **07.05.2025**
(21) Application number: 23831959.4
(22) Date of filing: 30.06.2023
(51) Int. Cl.: A61L 24/04, C08L 67/03, C08J 3/12

(54) **MEDICAL COMPOSITION**

(30) Priority: 30.06.2022 KR 20220080776; 29.06.2023 KR 20230084262
(71) Applicant: CJ CheilJedang Corporation, Seoul 04560 (KR); POSTECH Research and Business Development Foundation, Pohang-si, Gyeongsangbuk-do 37673 (KR)
(72) Inventor: SHIM, Yoo-Kyoung, Seoul 04560 (KR); LEE, Eun-Hye, Seoul 04560 (KR); YOON, Ki Chull, Seoul 04560 (KR); HWANG, Dong Soo, Pohang-si, Gyeongsangbuk-do 37673 (KR); YI, Gi-Ra, Pohang-si, Gyeongsangbuk-do 37673 (KR); OSMAN, Asila Ahmed Mohmmed, Pohang-si, Gyeongsangbuk-do 37673 (KR); JANG, Sookyeong, Pohang-si, Gyeongsangbuk-do 37673 (KR); LIN, Enhui, Pohang-si, Gyeongsangbuk-do 37673 (KR); PARK, Seong Hun, Pohang-si, Gyeongsangbuk-do 37673 (KR)
(74) Representative: Dehns
(86) International application number: PCT/KR2023/009252
(87) International publication number: WO 2024/005601

(57) **Abstract**

The present disclosure relates to a medical composition containing polyhydroxyalkanoate (PHA). The medical composition can exhibit excellent biodegradability, biocompatibility, adhesion to biological tissues, wound healing efficacy, and hemostatic efficacy. Therefore, the medical composition according to the present disclosure can be used for hemostasis, wound healing, tissue adhesion, or inhibition of bacterial infection.

## Description

### Technical Field

The present disclosure relates to a medical composition applicable to human or animal biological tissue and to a process for preparing the same.

### Background Art

When bleeding occurs due to injury in daily life or industrial settings, a surgical operation is performed to prevent excessive bleeding at the wound site, along with quick and safe emergency hemostasis. In such a surgical operation, effective hemostasis and suturing of the wound site can reduce the patient's bleeding and blood transfusion and promote the patient's recovery; thus, the treatment must be performed well.

Conventionally, medical equipment such as gauze, bandages, surgical sutures, staplers, or electricity and lasers have been used for the treatment of hemostasis and suturing. However, their use had the problem of causing side effects such as damage or infection to the wound site when the patient's biological tissue (e.g., skin) was fragile.

To solve this problem, hemostatic agents or medical adhesives have been developed. When the hemostatic agent or medical adhesive is applied to a bleeding wound, it exhibits physical, chemical, or bioactive properties to primarily prevent bleeding and help restore the wound site. Further, since it is applied to the wound site as coated, it can minimize damage or infection to the wound site even if the patient's biological tissue is fragile.

However, hemostatic agents or medical adhesives currently developed have limitations in that they do not exhibit the adhesive strength required for suturing the wound site or cause an immune response, resulting in poor biocompatibility.

### [Prior Art Document]

### [Patent Document]

(Patent Document 1) Korean Laid-open Patent Publication No. 2013-0055847

### Disclosure of Invention

### Technical Problem

The present disclosure aims to provide a medical composition that minimizes the occurrence of immune reactions, exhibits high adhesive strength to biological tissue, and is excellent in hemostatic efficacy, wound healing efficacy, inhibition of bacterial infection efficacy, and antibacterial efficacy.

In addition, the present disclosure aims to provide a process for preparing the medical composition.

### Solution to Problem

According to an aspect of the present disclosure to solve the above problem, there is provided a medical composition that comprises a polyhydroxyalkanoate (PHA) having a particle size of 10,000 nm or less and comprising a repeat unit derived from 3-hydroxybutyrate (3HB) in an amount of 40% by weight or more based on the total weight.

In an embodiment, the medical composition may have a tissue adhesive strength of 15 kPa or more when measured at a relative humidity of 100% and room temperature.

In another embodiment, the polyhydroxyalkanoate (PHA) may further comprise a repeat unit derived from 4-hydroxybutyrate (4HB).

In another embodiment, the content of the repeat unit derived from 4-hydroxybutyrate (4HB) may be 0.1 to 60% by weight based on the total weight of the polyhydroxyalkanoate (PHA).

In another embodiment, the polyhydroxyalkanoate (PHA) may be a poly(3-hydroxybutyrate-co-4-hydroxybutyrate) copolymer.

In another embodiment, the polyhydroxyalkanoate (PHA) may have a molecular weight of 10,000 to 1,200,000 g/mole.

In another embodiment, the concentration of the polyhydroxyalkanoate (PHA) contained in the medical composition may be 0.1 to 75% (w/v).

In another embodiment, the medical composition may be used for hemostasis.

In another embodiment, the medical composition may be used for wound healing.

In another embodiment, the medical composition may be used for tissue adhesion.

In another embodiment, the medical composition may be used for inhibition of bacterial infection.

Meanwhile, according to another aspect of the present disclosure, there is provided a process for preparing a medical composition that comprises dissolving a polyhydroxyalkanoate (PHA) in a solvent to prepare a dispersed phase solution; preparing a continuous phase solution comprising a surfactant; passing the dispersed phase solution through a membrane having a pore size of 10,000 nm or less to form an emulsion in which dispersed phase particles are dispersed in the continuous phase solution; and solidifying the emulsion to obtain polyhydroxyalkanoate (PHA) particles with a particle size of 10,000 nm or less.

In an embodiment, the content of the polyhydroxyalkanoate (PHA) contained in the dispersed phase solution may be 0.01 to 5% by weight based on the total weight of the dispersed phase solution.

In another embodiment, the dispersed phase solution may pass through the membrane at a pressure of 2.5 to 320 kPa.

According to another aspect of the present disclosure, there is provided a process for preparing a medical composition that comprises dissolving a polyhydroxyalkanoate (PHA) in a solvent to prepare a dispersed phase solution; preparing a continuous phase solution comprising a surfactant; mixing the dispersed phase solution and the continuous phase solution to form a premix emulsion; injecting the premix emulsion into a high-pressure dispersion device (microfluidizer) to form an emulsion in which polyhydroxyalkanoate (PHA) particles with a particle size of 10,000 nm or less are dispersed; and solidifying the emulsion to obtain the polyhydroxyalkanoate (PHA) particles.

In an embodiment, the pressure applied to the premix emulsion by the high-pressure dispersion device may be 1 to 30 kpsi.

### Advantageous Effects of Invention

As the medical composition according to the present disclosure comprises a polyhydroxyalkanoate (PHA) having a particle size controlled to a specific range, it has excellent biodegradability and biocompatibility (low immunogenicity) and can exhibit strong adhesive strength to biological tissue.

Accordingly, the medical composition according to the present disclosure can be efficiently used for hemostasis, healing, or suturing of biological tissue of humans or animals. In particular, the medical composition according to the present disclosure exhibits strong adhesive strength even to biological tissue containing moisture (e.g., tissue with a wet surface or tissue in water), whereby it can exhibit excellent hemostatic efficacy when used for hemostasis of a wound in water.

In addition, the medical composition according to the present disclosure can be formulated in various forms depending on the characteristics of the application site of biological tissue. In particular, the medical composition according to the present disclosure comprises a polyhydroxyalkanoate (PHA), which can be mass-produced through a microbial system; thus, it can economically provide a formulated hemostatic agent or medical adhesive.

### Brief Description of Drawings

Figs. 1 and 2 are images (SEM) showing polyhydroxyalkanoate (PHA) particles prepared in Synthesis Examples 1 to 5 and 10 to 15, respectively, according to the present disclosure.
Figs. 3 and 4 show the results of analyzing the changes in size and particle size distribution of polyhydroxyalkanoate (PHA) particles using a light scattering analysis method in Test Example 2 according to the present disclosure.
Figs. 5 and 6 show the results of measuring the expression levels of inflammatory cytokine factors (TNF-α and IL-6) in Test Example 3 according to the present disclosure.
Fig. 7 shows the results of evaluating the adhesion performance of the medical composition to pig skin in a 100% wet environment in Test Example 4 according to the present disclosure.
Fig. 8 shows the results of evaluating the adhesion performance of the medical composition to a hydrogel in Test Example 5 according to the present disclosure.
Fig. 9 is an image confirming the wound site over time in Test Example 6 according to the present disclosure.
Fig. 10 shows the results of calculating the changes in the wound site using the Image J program in Test Example 6 according to the present disclosure.
Fig. 11 is an image confirming the skin tissue and blood vessels of the wound site in Test Example 6 according to the present disclosure.
Figs. 12 and 13 are images confirming the wound site over time in Test Example 7 according to the present disclosure.
Figs. 14 to 18 are images showing the results of genetic analysis in Test Example 8 according to the present disclosure.
Fig. 19 shows the results of evaluating the hemostasis efficacy of the medical composition in Test Example 9 according to the present disclosure.
Fig. 20 shows the results of evaluating the hemostasis efficacy of the medical composition in Test Example 10 according to the present disclosure.
Fig. 21 shows the results of evaluating the inhibition of bacterial infection efficacy of the medical composition in Test Example 11 according to the present disclosure.

### Best Mode for Carrying out the Invention

Hereinafter, the present disclosure will be described with reference to embodiments. Here, the present disclosure is not limited to the disclosures given below, but it may be modified into various forms as long as the gist of the invention is not changed.

In the present specification, in the case where an element is mentioned to be formed, connected, or combined on or under another element, it means all of the cases where one element is directly, or indirectly through another element, formed, connected, or combined with another element. In addition, it should be understood that the criterion for the terms on and under of each component may vary depending on the direction in which the object is observed.

In the present specification, the term "comprising" is intended to specify a particular characteristic, region, step, process, element, and/or component. It does not exclude the presence or addition of any other characteristic, region, step, process, element and/or component, unless specifically stated to the contrary.

All numbers and expressions related to the quantities of components, reaction conditions, and the like used herein are to be understood as being modified by the term "about" unless otherwise indicated.

The present disclosure relates to a biocompatible composition comprising biocompatible polymer particles. Specifically, the present disclosure relates to a medical composition that can bond biological tissue and exhibit hemostatic efficacy, wound healing efficacy, inhibition of bacterial infection efficacy, and antibacterial efficacy for biological tissue, which will be described in detail as follows.

### Composition of the medical composition

The medical composition according to an embodiment of the present disclosure comprises a polyhydroxyalkanoate (PHA) having a particle size controlled to a specific range. Specifically, the medical composition according to an embodiment of the present disclosure may be one in which polyhydroxyalkanoate (PHA) particles having a particle size in a specific range are dispersed in a common solvent or solution.

The polyhydroxyalkanoate (PHA) has a particle size (average particle size) of 10,000 nm or less. Specifically, the particle size of the polyhydroxyalkanoate (PHA) may be 9,000 nm or less, 8,000 nm or less, 7,000 nm or less, 6,000 nm or less, 5,000 nm or less, 3,000 nm or less, 1,000 nm or less, 700 nm or less, 500 nm or less, 300 nm or less, or 200 nm or less. For example, the polyhydroxyalkanoate (PHA) may be particles having a particle size (average particle size) of 10 to 10,000 nm, 10 to 9,000 nm, 13 to 8,500 nm, 13 to 6,500 nm, 15 to 5,000 nm, 15 to 4,000 nm, 18 to 3,500 nm, 18 to 2,000 nm, 20 to 1,500 nm, 23 to 1,000 nm, 23 to 950 nm, 25 to 800 nm, 25 to 700 nm, 28 to 600 nm, 28 to 500 nm, 30 to 400 nm, 30 to 300 nm, 50 to 290 nm, 60 to 285 nm, 70 to 280 nm, 90 to 275 nm, 100 to 270 nm, 110 to 260 nm, 120 to 250 nm, 130 to 240 nm, 140 to 230 nm, or 150 to 260 nm. As the particle size of the polyhydroxyalkanoate (PHA) is within the above range, the medical composition according to the present disclosure can be applied uniformly (at high density) to biological tissue to maximize the adhesive area and adhesive strength. In particular, as the particle size of the polyhydroxyalkanoate (PHA) is controlled, the surface area of the polyhydroxyalkanoate (PHA) increases, which can enhance the interaction with biological tissue. As the present disclosure satisfies the above, it is possible to optimize the hemostatic effect and/or adhesive function of the medical composition. In addition, as the particle size of the polyhydroxyalkanoate (PHA) is within the above range, the medical composition according to the present disclosure can efficiently penetrate into wounded biological tissue (living tissue cells) to serve as a nutrient. As a result, the medical composition according to the present disclosure can have excellent wound healing efficacy.

The polyhydroxyalkanoate (PHA) may have a particle size deviation within ±0.1 µm. Specifically, the particle size deviation may be within ±0.09 µm, within ±0.07 µm, within ±0.05 µm, or within ±0.03 µm.

The particle size and particle size deviation of the polyhydroxyalkanoate (PHA) may refer to values measured using a nano particle size analyzer. Specifically, the particle size and particle size deviation of the polyhydroxyalkanoate (PHA) may be measured by dynamic light scattering (DLS) at a temperature of 25°C and a measurement angle of 175° using Zetasizer Nano ZS (manufacturer: Marven), which is a nano particle size analyzer. Here, the value of the peak derived through the polydispersity index (PDI) in the confidence interval of 0.5 may be defined as the particle size of the polyhydroxyalkanoate (PHA).

The polyhydroxyalkanoate (PHA) may have a polydispersity index (PDI) of 1 or less, specifically, 0.001 to 1, 0.003 to 0.9, 0.005 to 0.8, or 0.005 to 0.6.

As the particle size deviation and polydispersity index of the polyhydroxyalkanoate (PHA) are each within the above range, the dispersibility of the polyhydroxyalkanoate (PHA) particles and the processability of the medical composition can be enhanced.

The polyhydroxyalkanoate (PHA) comprises a repeat unit (a 3HB repeat unit) derived from 3-hydroxybutyrate (3HB). The content of the 3HB repeat unit may be 40% by weight or more, specifically, 40 to 99.9% by weight, 42 to 99.5% by weight, 45 to 99% by weight, 46 to 98.5% by weight, 47 to 98% by weight, 48 to 97% by weight, 50 to 96% by weight, 51 to 95% by weight, 52 to 94.5% by weight, 55 to 94% by weight, 60 to 93.5% by weight, 65 to 93% by weight, 70 to 93% by weight, 75 to 92.5% by weight, 80 to 92% by weight, or 82 to 91.5% by weight, based on the total weight of the polyhydroxyalkanoate (PHA).

The polyhydroxyalkanoate (PHA) may further comprise a repeat unit derived from at least one selected from the group consisting of 4-hydroxybutyrate (4HB), 3-hydroxypropionate (3HP), 3-hydroxyhexanoate (3HH), 3-hydroxyvalerate (3HV), 4-hydroxyvalerate (4HV), 5-hydroxyvalerate (5HV), and 6-hydroxyhexanoate (6HH).

Specifically, the polyhydroxyalkanoate (PHA) may further comprise a repeat unit (a 4HB repeat unit) derived from 4-hydroxybutyrate (4HB). As the polyhydroxyalkanoate (PHA) comprises a 4HB repeat unit, the biodegradability, biocompatibility, hemostatic efficacy, wound healing efficacy, and adhesive performance of the medical composition can be enhanced.

The content of the 4HB repeat unit may be 0.1 to 60% by weight, specifically, 0.5 to 58% by weight, 1 to 55% by weight, 1.5 to 54% by weight, 2 to 53% by weight, 3 to 52% by weight, 4 to 50% by weight, 5 to 49% by weight, 6 to 48% by weight, 6 to 45% by weight, 6.5 to 40% by weight, 7 to 35% by weight, 7 to 30% by weight, 7.5 to 25% by weight, 8 to 20% by weight, or 8.5 to 18% by weight, based on the total weight of the polyhydroxyalkanoate (PHA).

Specifically, the polyhydroxyalkanoate (PHA) may be a poly(3-hydroxybutyrate-co-4-hydroxybutyrate) copolymer. As a result, the biodegradability, biocompatibility, hemostatic efficacy, wound healing efficacy, and adhesive performance of the medical composition can be enhanced.

Meanwhile, the crystallinity of the polyhydroxyalkanoate (PHA) may be adjusted depending on the content of the 4HB repeat unit. It may be classified into semi-crystalline PHA (scPHA) and amorphous PHA (aPHA).

Specifically, the scPHA may have a content of a 4HB repeat unit of 0.1 to 30% by weight, 1 to 28% by weight, 3 to 26% by weight, 5 to 25% by weight, 6 to 23% by weight, 8 to 21% by weight, or 10 to 20% by weight. In addition, the aPHA may have a content of a 4HB repeat unit of 15 to 60% by weight, 20 to 58% by weight, 25 to 55% by weight, 35 to 53% by weight, 40 to 50% by weight, 43 to 49% by weight, or 45 to 48% by weight.

The polyhydroxyalkanoate (PHA) may be composed of the scPHA alone, the aPHA alone, or a mixture thereof.

The polyhydroxyalkanoate (PHA) may have a crystallinity of 90% or less, 85% or less, 80% or less, 75% or less, or 70% or less, as measured using a differential scanning calorimeter (DSC).

The polyhydroxyalkanoate (PHA) may have a molecular weight (weight average molecular weight) of 10,000 to 1,200,000 g/mole, specifically, 20,000 to 1,100,000 g/mole, 30,000 to 1,000,000 g/mole, 40,000 to 900,000 g/mole, 45,000 to 850,000 g/mole, 50,000 to 800,000 g/mole, 60,000 to 750,000 g/mole, 70,000 to 700,000 g/mole, 80,000 to 600,000 g/mole, 90,000 to 500,000 g/mole, 100,000 to 400,000 g/mole, 200,000 to 800,000 g/mole, or 300,000 to 700,000 g/mole. As the molecular weight of the polyhydroxyalkanoate (PHA) is within the above range, the adhesion performance of the medical composition to biological tissue can be enhanced.

The polyhydroxyalkanoate (PHA) may have a glass transition temperature (T_{g}) of -45 to 80°C, -35 to 50°C, -20 to 20°C, or -15 to 0°C. In addition, the crystallization temperature (T_{c}) of the polyhydroxyalkanoate (PHA) may not be measured or may be 60 to 120°C, 70 to 115°C, 75 to 110°C, or 80 to 105°C. In addition, the melting temperature (Tₘ) of the polyhydroxyalkanoate (PHA) may not be measured or may be 100 to 170°C, 105 to 160°C, 110 to 150°C, or 115 to 140°C.

The concentration of the polyhydroxyalkanoate (PHA) may be 0.1 to 75% (w/v) (0.1 to 75% by weight based on the total weight of the medical composition). Specifically, the concentration of the polyhydroxyalkanoate (PHA) contained in the medical composition according to an embodiment of the present disclosure may be 0.2 to 70% (w/v), 0.2 to 60% (w/v), 0.2 to 50% (w/v), 0.3 to 40% (w/v), 0.3 to 30% (w/v), 0.3 to 25% (w/v), 0.3 to 16% (w/v), 0.35 to 15.6% (w/v), 0.7 to 13% (w/v), 3 to 11% (w/v), or 6 to 9% (w/v). As the concentration of the polyhydroxyalkanoate (PHA) is within the above range, the hemostatic efficacy, wound healing efficacy, and adhesive performance of the medical composition can be significantly enhanced.

The medical composition comprising the polyhydroxyalkanoate (PHA) according to an embodiment of the present disclosure may have a tissue adhesive strength of 15 kPa or more when measured at a relative humidity of 100% and room temperature (e.g., 20±5°C, specifically 25°C). Specifically, the tissue adhesive strength of the medical composition according to an embodiment of the present disclosure may be 15 to 300 kPa, 18 to 200 kPa, 20 to 100 kPa, or 30 to 50 kPa. As the adhesive strength to biological tissue is within the above range, the medical composition can exhibit high adhesive performance not only to biological tissue without moisture but also to biological tissue with moisture (e.g., tissue with a wet surface or tissue in water).

The medical composition according to an embodiment of the present disclosure may further comprise a biocompatible polymer and/or an adhesive material other than the polyhydroxyalkanoate (PHA). The biocompatible polymer may specifically be catechol, caffeic acid, gallic acid, and tannin; polysaccharide-based polymers such as chitosan, hyaluronic acid, alginic acid, and dextran; or a protein-based polymer such as collagen and gelatin. The adhesive material may specifically be protamine, mussel adhesive protein, or squid sucker protein (suckerin).

In addition, the medical composition according to an embodiment of the present disclosure may further comprise a commonly known pharmacologically active substance. The pharmacologically active substance may specifically be an analgesic agent, antiinflammatory agent, antibacterial agent, or antifungal agent.

The medical composition according to an embodiment of the present disclosure may be formulated in various forms. Specifically, the medical composition according to an embodiment of the present disclosure may have a formulation such as powder, liquid, gel, or sheet (film).

### Use of the medical composition

The medical composition according to an embodiment of the present disclosure may be used in various applications. Specifically, the medical composition according to an embodiment of the present disclosure can be used for hemostasis, wound healing, bedsore healing, tissue adhesion, or inhibition of bacterial infection, which will be described in detail as follows.

### Hemostatic composition

The medical composition according to an embodiment of the present disclosure may be a hemostatic composition comprising a polyhydroxyalkanoate (PHA). The polyhydroxyalkanoate (PHA) has low immunogenicity, thereby having excellent biocompatibility, and it can effectively perform hemostasis. As the hemostatic composition according to an embodiment of the present disclosure comprises such a polyhydroxyalkanoate (PHA), it can effectively perform hemostasis of wounded biological tissue.

The polyhydroxyalkanoate (PHA) contained in the hemostatic composition according to an embodiment of the present disclosure has substantially the same composition and characteristics as those of the polyhydroxyalkanoate (PHA) described above in the "Composition of the medical composition."

Preferably, the polyhydroxyalkanoate (PHA) contained in the hemostatic composition may comprise a repeat unit derived from 4-hydroxybutyrate (4HB) in an amount of 0.1 to 30% by weight, 1 to 28% by weight, 3 to 26% by weight, 5 to 25% by weight, 6 to 23% by weight, or 10 to 20% by weight, based on the total weight of the polyhydroxyalkanoate (PHA), and it may have a molecular weight of 30,000 to 1,000,000 g/mole, 40,000 to 900,000 g/mole, 45,000 to 850,000 g/mole, or 50,000 to 800,000 g/mole. In addition, the polyhydroxyalkanoate (PHA) contained in the hemostatic composition may be particles having a particle size (average particle size) of 25 to 700 nm, 25 to 600 nm, 25 to 500 nm, 30 to 400 nm, 30 to 300 nm, 50 to 250 nm, 50 to 230 nm, 70 to 220 nm, 100 to 210 nm, 120 to 200 nm, 130 to 190 nm, 140 to 180 nm, or 150 to 180 nm.

The concentration of the polyhydroxyalkanoate (PHA) contained in the hemostatic composition according to an embodiment of the present disclosure may be 0.1 to 25% (w/v) (0.1 to 25% (w/v) based on the total weight of the hemostatic composition), specifically, 0.3 to 16% (w/v), 0.5 to 15% (w/v), 0.7 to 13% (w/v), 1 to 10% (w/v), 2 to 9% (w/v), 3 to 9% (w/v), or 6 to 8% (w/v). As the concentration of the polyhydroxyalkanoate (PHA) is within the above range, the hemostatic efficacy of the hemostatic composition can be significantly enhanced.

The hemostatic composition according to an embodiment of the present disclosure may comprise a common solvent or solution capable of uniformly dispersing the polyhydroxyalkanoate (PHA) while it is harmless to the living body (human body). The solvent or solution is not particularly limited, but it specifically includes purified water, saline solution, or a PBS buffer solution.

In addition, the hemostatic composition according to an embodiment of the present disclosure may optionally further comprise the above-described biocompatible polymer, the above-described adhesive material, and the above-described pharmacologically active substance.

### Wound healing composition

The medical composition according to an embodiment of the present disclosure may be a wound healing composition comprising the polyhydroxyalkanoate (PHA) as described above. The polyhydroxyalkanoate (PHA) is a nutrient that allows wounded biological tissue (living tissue cells) to recover quickly, thereby acting to increase the speed of recovery of the wounded biological tissue. As the wound healing composition according to an embodiment of the present disclosure comprises such a polyhydroxyalkanoate (PHA), it can efficiently perform healing (treatment) of wounded biological tissue.

The polyhydroxyalkanoate (PHA) contained in the wound healing composition according to an embodiment of the present disclosure has substantially the same composition and characteristics as those of the polyhydroxyalkanoate (PHA) described above in the "Composition of the medical composition."

Preferably, the polyhydroxyalkanoate (PHA) contained in the wound healing composition may comprise a repeat unit derived from 4-hydroxybutyrate (4HB) in an amount of 0.1 to 30% by weight, 1 to 28% by weight, 3 to 26% by weight, 5 to 25% by weight, 6 to 23% by weight, or 10 to 20% by weight, based on the total weight of the polyhydroxyalkanoate (PHA), and it may have a molecular weight of 30,000 to 1,000,000 g/mole, 40,000 to 900,000 g/mole, 45,000 to 850,000 g/mole, or 50,000 to 800,000 g/mole. In addition, the polyhydroxyalkanoate (PHA) contained in the wound healing composition may be particles having a particle size (average particle size) of 25 to 700 nm, 25 to 600 nm, 25 to 500 nm, 30 to 400 nm, 30 to 300 nm, 50 to 250 nm, 50 to 230 nm, 70 to 220 nm, 100 to 210 nm, 120 to 200 nm, 130 to 190 nm, 140 to 180 nm, or 150 to 180 nm.

The concentration of the polyhydroxyalkanoate (PHA) contained in the wound healing composition according to an embodiment of the present disclosure may be 0.1 to 25% (w/v) (0.1 to 25% (w/v) based on the total weight of the wound healing composition), specifically, 0.3 to 16% (w/v), 0.5 to 15% (w/v), 0.7 to 13% (w/v), 1 to 12% (w/v), 2 to 11% (w/v), 3 to 10% (w/v), 5 to 9% (w/v), or 6 to 8% (w/v). As the concentration of the polyhydroxyalkanoate (PHA) is within the above range, the wound healing efficacy of the wound healing composition can be significantly enhanced.

The wound healing composition according to an embodiment of the present disclosure may comprise a common solvent or solution capable of uniformly dispersing the polyhydroxyalkanoate (PHA) while it is harmless to the living body (human body). The solvent or solution is not particularly limited, but it specifically includes purified water, saline solution, or a PBS buffer solution.

In addition, the wound healing composition according to an embodiment of the present disclosure may optionally further comprise the above-described biocompatible polymer, the above-described adhesive material, and the above-described pharmacologically active substance.

### Tissue adhesion composition

The medical composition according to an embodiment of the present disclosure may be a tissue adhesion composition comprising the polyhydroxyalkanoate (PHA) as described above. The polyhydroxyalkanoate (PHA) has various functional groups (e.g., OH groups); thus, even when applied to wet biological tissue as well as biological tissue without moisture (e.g., skin tissue), it can act to strongly bind (adhere) it to other biological tissue. As the tissue adhesion composition according to an embodiment of the present disclosure comprises such a polyhydroxyalkanoate (PHA), it can efficiently perform the suturing (adhesion) of wounded biological tissue.

The polyhydroxyalkanoate (PHA) contained in the tissue adhesion composition according to an embodiment of the present disclosure has substantially the same composition and characteristics as those of the polyhydroxyalkanoate (PHA) described above in the "Composition of the medical composition."

Preferably, the polyhydroxyalkanoate (PHA) contained in the tissue adhesion composition may comprise a repeat unit derived from 4-hydroxybutyrate (4HB) in an amount of 0.1 to 30% by weight, 1 to 28% by weight, 3 to 26% by weight, 4 to 25% by weight, 5 to 23% by weight, 6 to 20% by weight, 7 to 18% by weight, or 8 to 19% by weight, based on the total weight of the polyhydroxyalkanoate (PHA), and it may have a molecular weight of 100,000 to 1,000,000 g/mole, 150,000 to 900,000 g/mole, 200,000 to 800,000 g/mole, or 300,000 to 700,000 g/mole. In addition, the polyhydroxyalkanoate (PHA) contained in the tissue adhesion composition may be particles having a particle size (average particle size) of 25 to 700 nm, 25 to 650 nm, 25 to 600 nm, 30 to 550 nm, 30 to 500 nm, 50 to 450 nm, 50 to 400 nm, 70 to 350 nm, 100 to 300 nm, 110 to 290 nm, 120 to 280 nm, 130 to 270 nm, or 140 to 260 nm.

The concentration of the polyhydroxyalkanoate (PHA) contained in the tissue adhesion composition according to an embodiment of the present disclosure may be 0.1 to 25% (w/v) (0.1 to 25% (w/v) based on the total weight of the tissue adhesion composition), specifically, 0.3 to 16% (w/v), 0.5 to 15% (w/v), 0.7 to 14% (w/v), 1 to 13% (w/v), 2 to 12% (w/v), 3 to 11% (w/v), 4 to 10% (w/v), or 5 to 10% (w/v). As the concentration of the polyhydroxyalkanoate (PHA) is within the above range, the biological tissue adhesion performance of the tissue adhesion composition can be significantly enhanced.

The tissue adhesion composition according to an embodiment of the present disclosure may have a tissue (biological tissue) adhesive strength of 15 kPa or more when measured at a relative humidity of 100% and room temperature (e.g., 20±5°C, specifically 25°C). Specifically, it may be 15 to 300 kPa, 16 to 270 kPa, 17 to 250 kPa, 18 to 200 kPa, 19 to 150 kPa, 20 to 100 kPa, 25 to 80 kPa, 28 to 60 kPa, or 30 to 50 kPa. In addition, the tissue adhesion composition according to an embodiment of the present disclosure may have a tissue adhesive strength of 1 to 300 J/m² to a hydrogel. Specifically, it may be 2 to 280 J/m², 3 to 250 J/m², 5 to 230 J/m², 6 to 200 J/m², 7 to 180 J/m², 8 to 150 J/m², 9 to 100 J/m², 10 to 80 J/m², 13 to 70 J/m², 15 to 60 J/m², 17 to 50 J/m², or 20 to 45 J/m². As the adhesive strength is within the above range, the tissue adhesion composition can exhibit high adhesive performance not only to biological tissue without moisture but also to biological tissue with moisture (e.g., tissue with a wet surface or tissue in water).

The tissue adhesion composition according to an embodiment of the present disclosure may comprise a common solvent or solution capable of uniformly dispersing the polyhydroxyalkanoate (PHA) while it is harmless to the living body. The solvent or solution is not particularly limited, but it specifically includes purified water, saline solution, or a PBS buffer solution.

In addition, the tissue adhesion composition according to an embodiment of the present disclosure may optionally further comprise the above-described biocompatible polymer, the above-described adhesive material, and the above-described pharmacologically active substance.

Composition for inhibiting bacterial infection (antibacterial or sterilizing

### composition)

The medical composition according to an embodiment of the present disclosure may be a composition for inhibiting bacterial infection comprising a polyhydroxyalkanoate (PHA). The polyhydroxyalkanoate (PHA) can act to inhibit the reproduction and metabolic function of germs (e.g., bacteria and the like). As the composition for inhibiting bacterial infection according to an embodiment of the present disclosure comprises such a polyhydroxyalkanoate (PHA), it can efficiently suppress bacterial infection in wounded biological tissue or tissue that has been subjected to surgery.

The polyhydroxyalkanoate (PHA) contained in the composition for inhibiting bacterial infection according to an embodiment of the present disclosure has substantially the same composition and characteristics as those of the polyhydroxyalkanoate (PHA) described above in the "Composition of the medical composition."

Preferably, the polyhydroxyalkanoate (PHA) contained in the composition for inhibiting bacterial infection may comprise a repeat unit derived from 4-hydroxybutyrate (4HB) in an amount of 0.1 to 30% by weight, 1 to 28% by weight, 3 to 26% by weight, 5 to 25% by weight, 6 to 23% by weight, or 10 to 20% by weight, based on the total weight of the polyhydroxyalkanoate (PHA), and it may have a molecular weight of 30,000 to 1,000,000 g/mole, 40,000 to 900,000 g/mole, 45,000 to 850,000 g/mole, or 50,000 to 800,000 g/mole. In addition, the polyhydroxyalkanoate (PHA) contained in the composition for inhibiting bacterial infection may be particles having a particle size (average particle size) of 25 to 700 nm, 25 to 600 nm, 25 to 500 nm, 30 to 400 nm, 30 to 300 nm, 50 to 250 nm, 50 to 230 nm, 70 to 220 nm, 100 to 210 nm, 120 to 200 nm, 130 to 190 nm, 140 to 180 nm, or 150 to 180 nm.

The concentration of the polyhydroxyalkanoate (PHA) contained in the composition for inhibiting bacterial infection according to an embodiment of the present disclosure may be 0.1 to 75% (w/v) (0.1 to 75% (w/v) based on the total weight of the composition for inhibiting bacterial infection), specifically, 5 to 75% (w/v), 10 to 75% (w/v), 15 to 70% (w/v), 20 to 70% (w/v), 25 to 65% (w/v), 30 to 65% (w/v), or 35 to 55% (w/v). As the concentration of the polyhydroxyalkanoate (PHA) is within the above range, the inhibition of bacterial infection efficacy of the composition for inhibiting bacterial infection can be significantly enhanced.

The composition for inhibiting bacterial infection according to an embodiment of the present disclosure may comprise a common solvent or solution capable of uniformly dispersing the polyhydroxyalkanoate (PHA) while it is harmless to the living body (human body). The solvent or solution is not particularly limited, but it specifically includes purified water, saline solution, or a PBS buffer solution.

In addition, the composition for inhibiting bacterial infection according to an embodiment of the present disclosure may optionally further comprise the above-described biocompatible polymer, the above-described adhesive material, and the above-described pharmacologically active substance.

### Process for preparing a medical composition

In the present disclosure, a medical composition can be prepared through a procedure of granulating a polyhydroxyalkanoate (PHA) to have a particle size that can exhibit optimal biocompatibility.

Specifically, the process for preparing a medical composition according to an embodiment of the present disclosure comprises dissolving a polyhydroxyalkanoate (PHA) in a solvent to prepare a dispersed phase solution (S-1); preparing a continuous phase solution comprising a surfactant (S-2); passing the dispersed phase solution through a membrane having a pore size of 10,000 nm or less to form an emulsion in which dispersed phase particles are dispersed in the continuous phase solution (S-3); and solidifying the emulsion to obtain polyhydroxyalkanoate (PHA) particles with a particle size of 10,000 nm or less (S-4).

Step S-1 is a step of dissolving (dispersing) a polyhydroxyalkanoate (PHA), an initial raw material, in a solvent to prepare a dispersed phase solution in which the polyhydroxyalkanoate (PHA) is dispersed.

The polyhydroxyalkanoate (PHA) used as an initial raw material may be obtained by cell disruption of microorganisms using a mechanical method or a physical method, or it may be obtained by cell disruption of microorganisms using a non-mechanical method or a chemical method.

The solvent may not be particularly limited as long as it can dissolve the polyhydroxyalkanoate (PHA) as an initial raw material. Specifically, it may be at least one selected from the group consisting of chloroform, chloroethane, dichloromethane, dichloroethane, butyl acetate, and trichloroethane.

Meanwhile, dissolution of the polyhydroxyalkanoate (PHA) may be carried out by stirring at 40 to 50°C for 45 to 55 hours so that the polyhydroxyalkanoate (PHA) can be evenly dissolved in the solvent.

The dispersed phase solution prepared through this procedure may comprise 0.01 to 5% by weight of a polyhydroxyalkanoate (PHA) as an initial raw material. Specifically, the dispersed phase solution prepared through this procedure may comprise a polyhydroxyalkanoate (PHA) in an amount of 0.02 to 4.5% by weight, 0.05 to 4% by weight, 0.08 to 3.5% by weight, 0.1 to 3% by weight, 0.2 to 2.5% by weight, 0.35 to 2% by weight, 0.4 to 1.5% by weight, 0.5 to 1.3% by weight, 0.6 to 1.2% by weight, 0.7 to 1% by weight, or 0.75 to 0.9% by weight, based on the total weight of the dispersed phase solution. As the content of the polyhydroxyalkanoate (PHA) contained in the dispersed phase solution is within the above range, polyhydroxyalkanoate (PHA) particles having a particle size of 10,000 nm or less and a uniform shape (spherical shape) can be obtained.

Step S-2 is a step of preparing a continuous phase solution comprising a surfactant (emulsifier). Specifically, the continuous phase solution may comprise a surfactant and an aqueous solvent.

The surfactant may specifically be at least one selected from the group consisting of sodium dodecyl sulfate, sodium lauryl sulfate, sodium laureth sulfate, sodium stearate, sodium cocoyl glycinate, and perfluorooctane sulfonate.

The aqueous solvent may be water, distilled water, deionized water, pure water, or ultrapure water.

In the preparation of the continuous phase solution, the stirring speed may be 1 to 800 rpm, 50 to 600 rpm, or 200 to 500 rpm. As the stirring speed of the continuous phase solution is within the above range, polyhydroxyalkanoate (PHA) particles having a uniform nano-level size can be obtained.

Step S-2 of preparing the continuous phase solution may be carried out simultaneously with step S-1, or it may be carried out before, or after, step S-1.

Step S-3 is a step of passing the dispersed phase solution prepared in step S-1 through a membrane having a pore size of 10,000 nm or less to form an emulsion in which dispersed phase particles (PHA dispersed phase particles) are dispersed in the continuous phase solution prepared in step S-2.

The membrane may specifically be an inorganic porous membrane (e.g., Shirasu porous glass (SPG)) and has a pore size of 10,000 nm or less, so that the polyhydroxyalkanoate (PHA) particles obtained can be made to have a particle size of 10,000 nm or less, which is the required particle size.

The pressure at which the dispersed phase solution passes through the membrane may be 2.5 to 320 kPa, specifically, 50 to 320 kPa, 100 to 320 kPa, or 200 to 320 kPa. More specifically, the pressure at which the dispersed phase solution passes through the membrane may be adjusted depending on the size of the pores. The larger the pores, the lower the pressure may be used. As the pressure is within the above range, polyhydroxyalkanoate (PHA) particles having a uniform shape (spherical shape) can be obtained.

Step S-4 is a step of solidifying the emulsion comprising the dispersed phase particles to obtain polyhydroxyalkanoate (PHA) particles with a particle size of 10,000 nm or less. Solidification of the emulsion may refer to removing the solvent used to dissolve the polyhydroxyalkanoate (PHA) in step S-1. Solidification of the emulsion may be carried out through natural drying, vacuum drying using an oven, or drying using a rotary evaporator. Solidification of the emulsion can produce a product comprising the polyhydroxyalkanoate (PHA) particles (e.g., an aqueous dispersion in which the PHA particles with a particle size of 10,000 nm or less are dispersed).

Meanwhile, the process for preparing a medical composition according to another embodiment of the present disclosure comprises dissolving a polyhydroxyalkanoate (PHA) in a solvent to prepare a dispersed phase solution (S-1'); preparing a continuous phase solution comprising a surfactant (S-2'); mixing the dispersed phase solution and the continuous phase solution to form a premix emulsion (S-3'); injecting the premix emulsion into a high-pressure dispersion device (microfluidizer) to form an emulsion in which polyhydroxyalkanoate (PHA) particles with a particle size of 10,000 nm or less are dispersed (S-4'); and solidifying the emulsion to obtain the polyhydroxyalkanoate (PHA) particles with a particle size of 10,000 nm or less (S-5').

Steps S-1' and S-2' each have substantially the same configuration and characteristics as those of steps S-1 and S-2. Thus, a detailed description thereof will be omitted.

Step S-3' is a step of mixing the dispersed phase solution prepared in step S-1' and the continuous phase solution prepared in step S-2' to form a premix emulsion (A).

Mixing to form the premix emulsion (A) may be carried out using a conventional homogenizer. The mixing through the homogenizer may be carried out for 1 to 5 minutes at a stirring speed of 5,000 to 15,000 rpm.

Step S-4' is a step of injecting the premix emulsion (A) into a high-pressure dispersion device (microfluidizer) to form an emulsion (B) in which polyhydroxyalkanoate (PHA) particles (dispersed phase particles) with a particle size of 10,000 nm or less are dispersed.

The high-pressure dispersion device (high-pressure dispersion emulsification device) is a device that applies pressure to the premix emulsion (A) to split the droplets of the premix emulsion (A) into smaller pieces. A typical high-pressure dispersion device may be used.

The pressure applied to the premix emulsion (A) by the high-pressure dispersion device may be 1 to 30 kpsi. Specifically, it may be 3 to 25 kpsi, 5 to 20 kpsi, or 9 to 15 kpsi. More specifically, the operating pressure of the high-pressure dispersion device may be adjusted depending on the content of the polyhydroxyalkanoate (PHA) contained in the dispersed phase solution. The higher the content of polyhydroxyalkanoate (PHA), the higher the pressure it may be operated. As the pressure is within the above range, polyhydroxyalkanoate (PHA) particles having a uniform shape (spherical shape) can be obtained.

Step S-5' is a step of solidifying the emulsion (B) comprising the polyhydroxyalkanoate (PHA) particles (dispersed phase particles) to obtain the polyhydroxyalkanoate (PHA) particles with a particle size of 10,000 nm or less. Solidification of the emulsion may refer to removing the solvent used to dissolve the polyhydroxyalkanoate (PHA) in step S-1'. Solidification of the emulsion (B) may be carried out through natural drying, vacuum drying using an oven, or drying using a rotary evaporator. Solidification of the emulsion (B) can produce a product comprising the polyhydroxyalkanoate (PHA) particles (e.g., an aqueous dispersion in which the PHA particles with a particle size of 10,000 nm or less are dispersed).

As polyhydroxyalkanoate (PHA) particles are prepared through a membrane emulsification method using a membrane or an emulsification method using a high-pressure dispersion device as described above, the present disclosure can provide polyhydroxyalkanoate (PHA) particles with a particle size of 10,000 nm or less and a uniform shape.

Meanwhile, the polyhydroxyalkanoate (PHA) particles (the product comprising the polyhydroxyalkanoate (PHA) particles) obtained through step S-4 or step S-5' may be subjected to an additional procedure such as sterilization. Thereafter, the polyhydroxyalkanoate (PHA) particles (the product comprising the polyhydroxyalkanoate (PHA) particles) themselves thus obtained may be used as the medical composition according to an embodiment of the present disclosure. In addition, the obtained polyhydroxyalkanoate (PHA) particles may be subjected to a procedure for forming a formulation such as powdering, liquefying, gelling, sheeting (filming), and the like, to be used as the medical composition according to an embodiment of the present disclosure.

### Mode for the Invention

Hereinafter, the present disclosure will be described in detail with reference to Examples, but the scope of the present disclosure is not limited to the Examples.

### <Preparation of PHA particles>

### Synthesis Example 1

PHA particles were prepared through membrane emulsification using a membrane. Specifically, a poly(3-hydroxybutyrate-co-4-hydroxybutyrate) copolymer ([P(3HB-co-4HB)], 4HB repeat unit: 17% by weight, molecular weight: 687,000 g/mole) was dissolved in chloroform to prepare 10 ml of a dispersed phase solution (PHA content: 0.4% by weight or less). In addition, 100 ml of a continuous aqueous solution comprising 0.3% by weight of sodium dodecyl sulfate as a surfactant, was prepared.

An IMK-40 (MCTech) device using an SPG (Shirasu-porous glass) membrane was used as an emulsification device, and the prepared dispersed phase solution and the continuous phase solution were used to induce the formation of an emulsion. In such an event, the pore size of the membrane was 300 nm, the pressure for passing through the membrane was controlled to 150 to 320 kPa, and the stirring speed was controlled to 100 to 300 rpm.

200 ml of the formed emulsion was poured into a container capable of maintaining an interface height of less than 10 mm, and the chloroform was slowly evaporated at room temperature for 48 hours to induce solidification of the emulsion. As a result, PHA particles (specifically, an aqueous dispersion containing PHA particles) with a particle size of 300 nm or less were obtained.

### Synthesis Examples 2 to 5

PHA particles (specifically, an aqueous dispersion containing PHA particles) were obtained through the same procedure as in Synthesis Example 1, respectively, except that a membrane having a pore size of 200 nm (Synthesis Example 2), 1 µm (Synthesis Example 3), 5 µm (Synthesis Example 4), or 10 µm (Synthesis Example 5) was used.

### Synthesis Examples 6 to 9

PHA particles (specifically, an aqueous dispersion containing PHA particles) were obtained through the same procedure as in Synthesis Example 1, respectively, except that the composition and molecular weight of the poly(3-hydroxybutyrate-co-4-hydroxybutyrate) copolymer dissolved in chloroform were adjusted as shown in Table 1 below.

### Synthesis Example 10

PHA particles were prepared through membrane emulsification using a membrane. Specifically, a poly(3-hydroxybutyrate-co-4-hydroxybutyrate) copolymer ([P(3HB-co-4HB)], 4HB repeat unit: 8.7% by weight, molecular weight: 390,000 g/mole) was dissolved in chloroform to prepare 10 ml of a dispersed phase solution (PHA content: 1.0% by weight or less). In addition, 100 ml of a continuous aqueous solution comprising 0.3% by weight of sodium dodecyl sulfate as a surfactant, was prepared.

An IMK-40 (MCTech) device using an SPG (Shirasu-porous glass) membrane was used as an emulsification device, and the prepared dispersed phase solution and the continuous phase solution were used to induce the formation of an emulsion. In such an event, the pore size of the membrane was 300 nm, the pressure for passing through the membrane was controlled to 150 to 320 kPa, and the stirring speed was controlled to 100 to 300 rpm.

200 ml of the formed emulsion was poured into a 1-liter round bottom flask, and the chloroform was evaporated at 57°C and 150 mbar for 4 hours to induce solidification of the emulsion. As a result, PHA particles (specifically, an aqueous dispersion containing PHA particles) with a particle size of 300 nm or less were obtained.

### Synthesis Examples 11 to 14

PHA particles (specifically, an aqueous dispersion containing PHA particles) were obtained through the same procedure as in Synthesis Example 10, respectively, except that a membrane having a pore size of 1 µm (Synthesis Example 11), 2 µm (Synthesis Example 12), 5 µm (Synthesis Example 13), or 10 µm (Synthesis Example 14) was used.

### Synthesis Examples 15 to 17

PHA particles (specifically, an aqueous dispersion containing PHA particles) were obtained through the same procedure as in Synthesis Example 10, respectively, except that the content (concentration) of the poly(3-hydroxybutyrate-co-4-hydroxybutyrate) copolymer dissolved in chloroform was adjusted as shown in Table 1 below.

### Synthesis Examples 18 to 20

PHA particles (specifically, an aqueous dispersion containing PHA particles) were obtained through the same procedure as in Synthesis Example 11, respectively, except that the content (concentration) of the poly(3-hydroxybutyrate-co-4-hydroxybutyrate) copolymer dissolved in chloroform was adjusted as shown in Table 1 below.

### Synthesis Examples 21 to 25

PHA particles (specifically, an aqueous dispersion containing PHA particles) were obtained through the same procedure as in Synthesis Example 10, respectively, except that the composition and molecular weight of the poly(3-hydroxybutyrate-co-4-hydroxybutyrate) copolymer dissolved in chloroform were adjusted as shown in Table 1 below.

### Synthesis Example 26

PHA particles were prepared through an emulsification method using a high-pressure dispersion device. Specifically, a poly(3-hydroxybutyrate-co-4-hydroxybutyrate) copolymer ([P(3HB-co-4HB)], 4HB repeat unit: 8.7% by weight, molecular weight: 390,000 g/mole) was dissolved in chloroform to prepare 25 ml of a dispersed phase solution (PHA content: 5% by weight). In addition, 500 ml of a continuous aqueous solution comprising 0.3% by weight of sodium dodecyl sulfate as a surfactant, was prepared.

The dispersed phase solution was added to the continuous phase aqueous solution and mixed for 2 minutes using a homogenizer (IKA ULTRA-TURRAX^{®} T 25 digital, IKA) set at 13,600 rpm to form a premix emulsion.

Subsequently, the premix emulsion was fed to a high-pressure dispersion device (LM20, Microfluidics), and pressure was applied to induce the formation of a homogenized emulsion. In such an event, the pressure was controlled to 10 kpsi.

200 ml of the formed emulsion was poured into a 1-liter round bottom flask, and the chloroform was evaporated at 57°C and 150 mbar for 4 hours to induce solidification of the emulsion. As a result, PHA particles (specifically, an aqueous dispersion containing PHA particles) with a particle size of 300 nm or less were obtained.

**[Table 1]**

| | Initial raw material PHA ([P(3HB-co-4HB)]) | | PHA content in the dispersed phase solution (% by weight) | Membrane pore size | Particle size of the obtained PHA |
|---|---|---|---|---|---|
| | 4HB repeat unit (% by weight) | Molecular weight (g/mole) | | | |
| Synthesis Ex. 1 | 17 | 687,000 | 0.334 | 300 nm | 202.5 nm |
| Synthesis Ex. 2 | 17 | 687,000 | 0.334 | 200 nm | 158.9 nm |
| Synthesis Ex. 3 | 17 | 687,000 | 0.334 | 1 µm | 791 nm |
| Synthesis Ex. 4 | 17 | 687,000 | 0.334 | 5 µm | 3,230 nm |
| Synthesis Ex. 5 | 17 | 687,000 | 0.334 | 10 µm | 6,170 nm |
| Synthesis Ex. 6 | 6 | 50,000 | 0.334 | 300 nm | 202.7 nm |
| Synthesis Ex. 7 | 16 | 257,000 | 0.334 | 300 nm | 213.9 nm |
| Synthesis Ex. 8 | 7 | 576,000 | 0.334 | 300 nm | 210.7 nm |
| Synthesis Ex. 9 | 48 | 800,000 | 0.334 | 300 nm | 249.6 nm |
| Synthesis Ex. 10 | 8.7 | 390,000 | 0.8 | 300 nm | 274.3 nm |
| Synthesis Ex. 11 | 8.7 | 390,000 | 0.8 | 1 µm | 913 nm |
| Synthesis Ex. 12 | 8.7 | 390,000 | 0.8 | 2 µm | 1,929 nm |
| Synthesis Ex. 13 | 8.7 | 390,000 | 0.8 | 5 µm | 4,737 nm |
| Synthesis Ex. 14 | 8.7 | 390,000 | 0.8 | 10 µm | 8,480 nm |
| Synthesis Ex. 15 | 8.7 | 390,000 | 0.1 | 300 nm | 197.7 nm |
| Synthesis Ex. 16 | 8.7 | 390,000 | 0.2 | 300 nm | 236 nm |
| Synthesis Ex. 17 | 8.7 | 390,000 | 0.4 | 300 nm | 260.2 nm |
| Synthesis Ex. 18 | 8.7 | 390,000 | 0.1 | 1 µm | 515.2 nm |
| Synthesis Ex. 19 | 8.7 | 390,000 | 0.2 | 1 µm | 600 nm |
| Synthesis Ex. 20 | 8.7 | 390,000 | 0.4 | 1 µm | 710.7 nm |
| Synthesis Ex. 21 | 6 | 50,000 | 0.8 | 300 nm | 284.8 nm |
| Synthesis Ex. 22 | 7 | 576,000 | 0.8 | 300 nm | 223.5 nm |
| Synthesis Ex. 23 | 16 | 257,000 | 0.8 | 300 nm | 247 nm |
| Synthesis Ex. 24 | 17 | 687,000 | 0.8 | 300 nm | 210.4 nm |
| Synthesis Ex. 25 | 48 | 800,000 | 0.8 | 300 nm | 237 nm |
| Synthesis Ex. 26 | 8.7 | 390,000 | 5 | - | 131.1 nm |

### Test Example 1: Observation of PHA particles

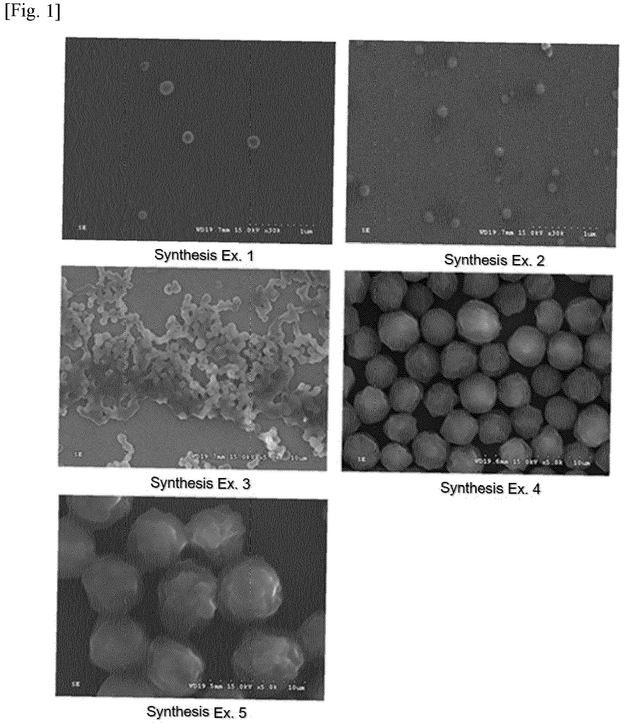

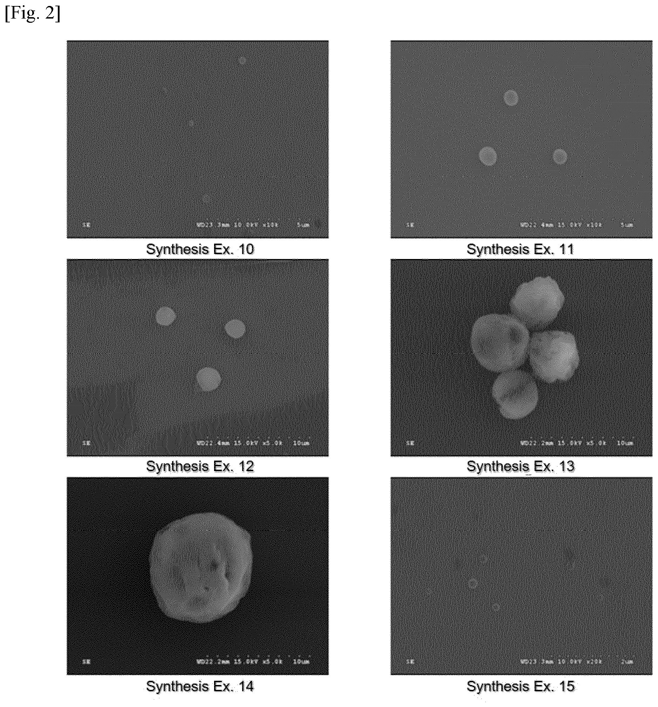

The PHA particles obtained in Synthesis Examples 1 to 5 and Synthesis Examples 10 to 15, respectively, were observed through scanning electron microscopy (SEM). The results are shown in Figs. 1 and 2.

Referring to Figs. 1 and 2, PHA particles having a uniform size and spherical shape were well prepared as the PHA particles were prepared using a membrane emulsification method.

### Test Example 2: Analysis of PHA particle distribution

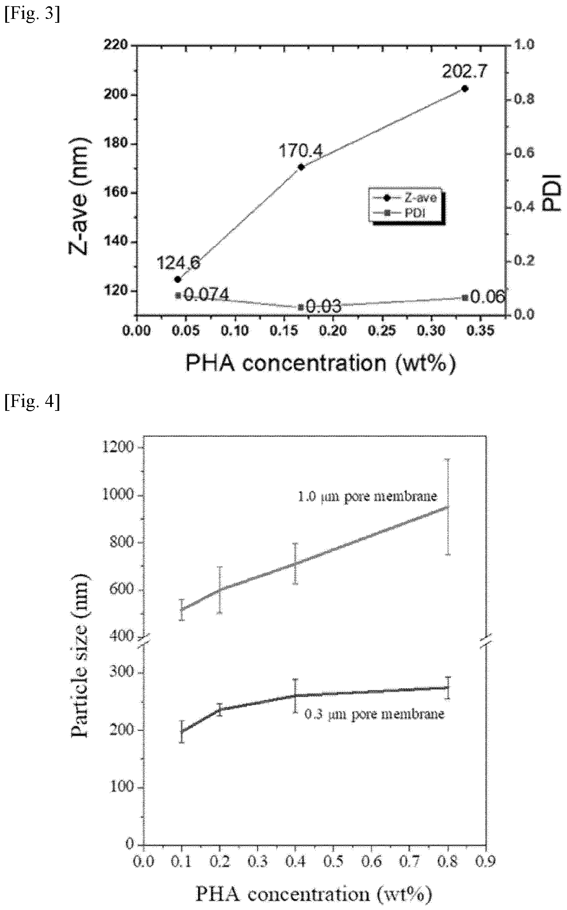

PHA particles were obtained through the same procedure as in Synthesis Example 1, except that the content (concentration) of the PHA was adjusted to 0.042 to 0.334% by weight when the dispersed phase solution was prepared. The size of the obtained PHA particles and the changes in its distribution were analyzed using light scattering analysis (Zetasizer Nano ZS, Malvern Instruments, U.K.). The results are shown in Fig. 3.

In addition, PHA particles were obtained through the same procedure as in Synthesis Examples 10 and 11, except that the content (concentration) of the PHA was adjusted to 0.1 to 0.8% by weight when the dispersed phase solution was prepared. The size of the obtained PHA particles and the changes in its distribution were analyzed using light scattering analysis (Zetasizer Nano ZS, Malvern Instruments, U.K.). The results are shown in Fig. 4.

Referring to Fig. 3, as the content of PHA (initial raw material) contained in the dispersed phase solution was within the range of 0.01 to 0.5% by weight, PHA particles with a particle diameter of 300 nm or less were well prepared. In addition, referring to Fig. 4, as the content of PHA (initial raw material) contained in the dispersed phase solution was within the range of 0.1 to 1% by weight, and as the pore size of the membrane was 0.3 µm, PHA particles with a particle diameter of 300 nm or less were well prepared.

### Test Example 3: Evaluation of PHA immunogenicity

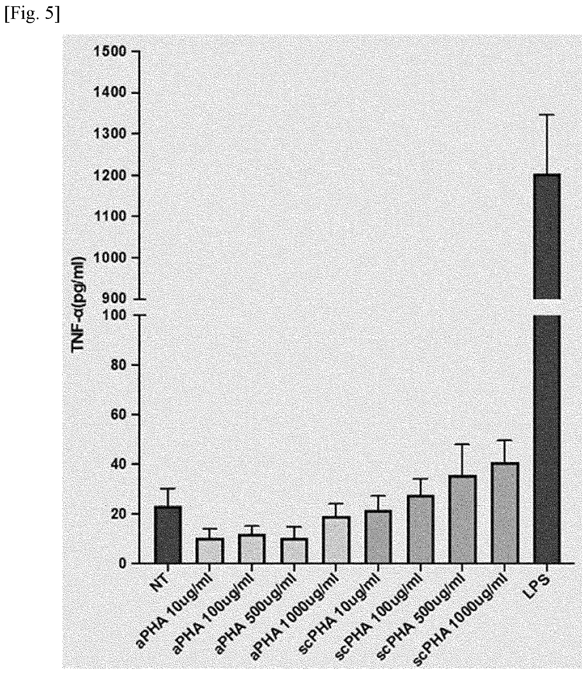

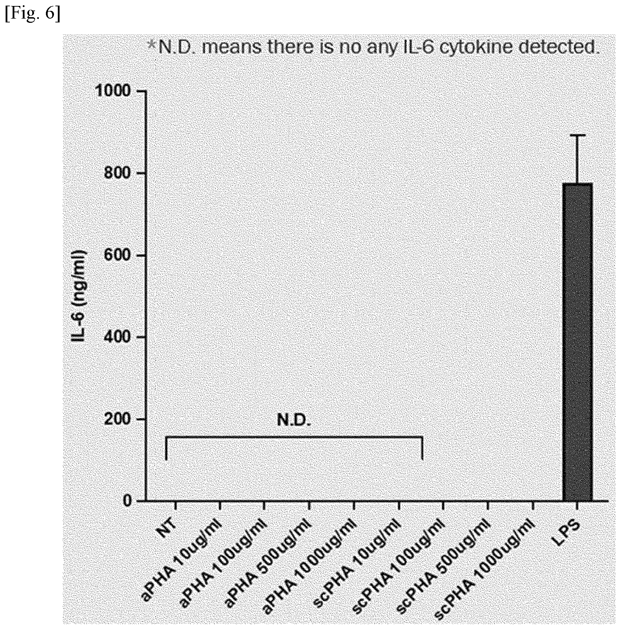

The semi-crystalline PHA of Synthesis Example 1 (scPHA, [P(3HB-co-4HB)] form, repeat unit derived from 4HB: 17% by weight, molecular weight: 687,000 g/mole) and the amorphous PHA of Synthesis Example 9 (aPHA, [P(3HB-co-4HB)] form, repeat unit derived from 4HB: 48% by weight, molecular weight: 800,000 g/mole) were each evaluated for immunogenicity. The results are shown in Figs. 5 and 6. Specifically, Raw 264.7 cells were suspended in DMEM (Dulbecco's modified eagle medium) containing 10% FBS (fetal bovine serum), which was distributed to a 48-well plate at a cell count of 1×10⁵ cells/ml and cultured for 12 days in a CO₂ incubator at 37°C. Thereafter, scPHA or aPHA dissolved (dispersed) in a 0.1 M PBS buffer (pH 7.4) was treated at concentrations of 10 µg/ml, 100 µg/ml, 500 µg/ml, and 1,000 µg/ml, respectively, and cultured for 24 hours. The cell culture medium after culturing was taken, and the contents of TNF-α and IL-6, as inflammatory cytokine factors, were measured using an ELISA kit. In such an event, LPS (Lipopolysaccharides from *Escherichia coli* O55:B5 (Cat# L2880, Sigma-Aldrich, St. Louis, MO, USA)) was used as a positive control, and a 0.1 M PBS buffer (pH 7.4) (NT) was used as a negative control.

Referring to Figs. 5 and 6, it can be observed that the expression of the inflammatory cytokine factors TNF-α and IL-6 was suppressed by the PHA according to the present disclosure, aPHA 10 µg/ml, aPHA 100 µg/ml, aPHA 500 µg/ml, aPHA 1,000 µg/ml, scPHA 10 µg/ml, scPHA 100 µg/ml, and scPHA 1,000 µg/ml. In particular, when aPHA 1,000 µg/ml and scPHA 1,000 µg/ml were compared with LPS (positive control) treated at the same concentration, it is confirmed that the PHA according to the present disclosure had a significantly lower expression of the inflammatory cytokine factors than that of LPS.

These results support that the PHA according to the present disclosure has low immunogenicity and excellent biocompatibility.

### <Preparation of a medical composition>

### Example 1

PHA particles with a particle size of 178 nm ([P(3HB-co-4HB)], 4HB repeat unit: 17% by weight, molecular weight: 687,000 g/mole) were obtained through the same procedure as in Synthesis Example 1, except that the pore size of the membrane was adjusted. The obtained PHA particles were dissolved (dispersed) in a 0.1 M PBS buffer (pH 7.4) at a concentration in the range of 0.3 to 16% (w/v) to prepare a composition.

### Example 2

PHA particles with a particle size of 259 nm ([P(3HB-co-4HB)], 4HB repeat unit: 17% by weight, molecular weight: 687,000 g/mole) were obtained through the same procedure as in Synthesis Example 1, except that the pore size of the membrane was adjusted. The obtained PHA particles were dissolved (dispersed) in a 0.1 M PBS buffer (pH 7.4) at a concentration in the range of 0.3 to 16% (w/v) to prepare a composition.

### Example 3

PHA particles with a particle size of 180 nm ([P(3HB-co-4HB)], 4HB repeat unit: 8.7% by weight, molecular weight: 390,000 g/mole) were obtained through the same procedure as in Synthesis Example 11, except that the pore size of the membrane was adjusted. The obtained PHA particles were subjected to a procedure to adjust the concentration to the range of 0.1 to 10% (w/v) to prepare a composition. In such an event, the concentration of PHA particles was adjusted by concentrating with a rotary evaporator or diluting with purified water.

### Example 4

A composition was prepared through the same procedure as in Example 3, except that PHA particles with a particle diameter of 580 nm ([P(3HB-co-4HB)], 4HB repeat unit: 8.7% by weight, molecular weight: 390,000 g/mole) were obtained.

### Example 5

A composition was prepared through the same procedure as in Example 3, except that PHA particles with a particle diameter of 850 nm ([P(3HB-co-4HB)], 4HB repeat unit: 8.7% by weight, molecular weight: 390,000 g/mole) were obtained.

### Example 6

A composition was prepared through the same procedure as in Example 3, except that PHA particles with a particle diameter of 4,740 nm ([P(3HB-co-4HB)], 4HB repeat unit: 8.7% by weight, molecular weight: 390,000 g/mole) were obtained.

### Example 7

A composition was prepared through the same procedure as in Example 3, except that PHA particles with a particle diameter of 8,190 nm ([P(3HB-co-4HB)], 4HB repeat unit: 8.7% by weight, molecular weight: 390,000 g/mole) were obtained.

### Comparative Example 1

Fibrin glue (Green Cross, Korea) (FG) was used.

### Test Example 4: Evaluation of adhesion performance 1

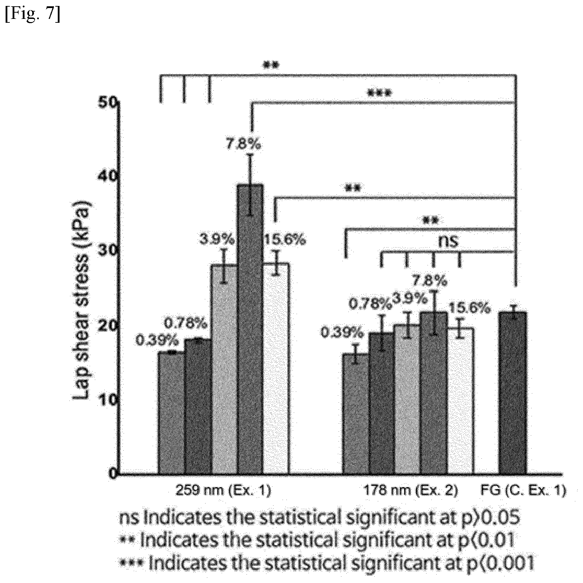

The adhesive strength of each composition to biological tissue was evaluated by measuring shear stress using a universal testing machine (Instron 5544, Norwood, MA, US). The results are shown in Fig. 7. Specifically, pig skin (Stellen Medical, USA) was cut into 10 mm × 10 mm pieces, placed in a 0.1 M PBS buffer (pH 7.4), and left at 37°C for 1 hour. Thereafter, the pork skin was attached to an aluminum bar having a size of 10 mm × 100 mm using an instant adhesive (3M). Then, the compositions prepared in Examples 1 and 2 and the fibrin glue of Comparative Example 1 were each applied to the surface of the pig skin as a specimen. Next, an aluminum bar with pig skins on which no specimen was applied was superposed thereon, and the two aluminum bars were then fixed using clips. The two fixed aluminum bars were incubated for 2 hours at a relative humidity of 100% and room temperature (RT). Then, the shear stress until the two aluminum bars were completely separated was measured at a crosshead speed of 10 mm/minute using a 10 kN load cell.

Referring to Fig. 7, it can be confirmed that the compositions in Examples 1 and 2 according to the present disclosure exhibit adhesive strength equal to, or better than, that of fibrin glue of Comparative Example 1. In particular, when the particle size of the PHA particles was 259 nm and the concentration of the PHA particles was 3 to 16% (w/v), significantly high adhesive strength was exhibited.

These results support that it is necessary to adjust the particle size of PHA particles to increase adhesive strength to biological tissue and that the medical composition (tissue adhesion composition) according to the present disclosure exhibits strong adhesive strength even in a moist environment.

### Test Example 5: Evaluation of adhesion performance 2

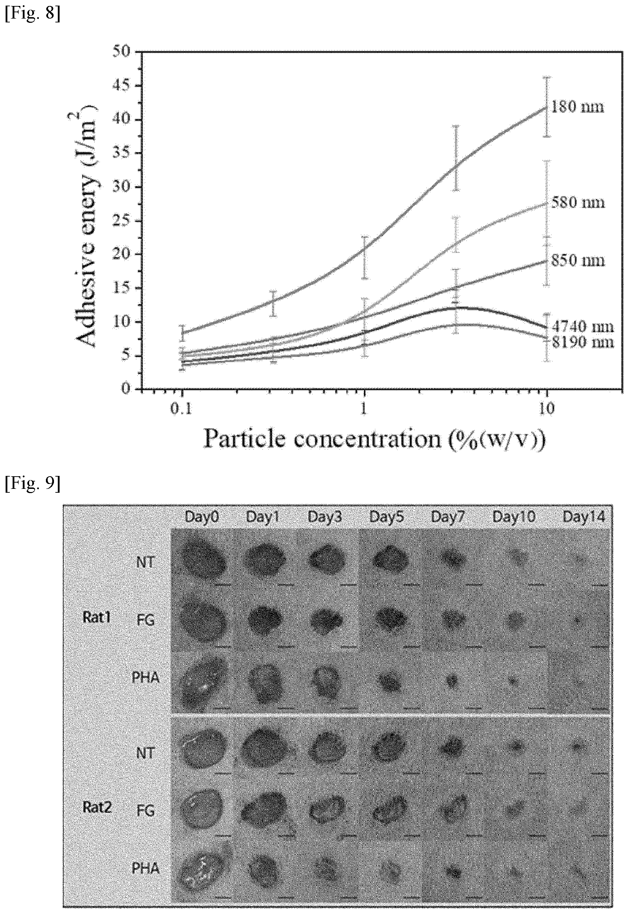

The adhesive strength of each composition to biological tissue was evaluated by measuring shear stress using a universal testing machine (QC-508E, Cometech, Taiwan). The results are shown in Fig. 8. Specifically, 27.6 mg of N,N'-methylene bis(acrylamide) and 4,920 mg of potassium persulfate were dissolved in 30 g of deionized water at 4°C. Then, 18.528 ml of N,N-dimethylacrylamide and 270 µl of N,N,N',N'-tetramethylethylenediamine were added thereto, followed by mixing thereof. This solution was placed in a mold with a width of 1.5 mm and crosslinked at 30°C for 16 hours to prepare a hydrogel. The prepared hydrogel was cut into a size of 5 mm × 30 mm, and 10 µl of each composition prepared in Examples 3 to 7 was applied as a specimen to the surface of the hydrogel in an area of 5 mm × 10 mm. Next, two hydrogel pieces coated with the specimen were superposed and left at room temperature (RT) for 4 hours. After 4 hours, the shear stress until the two hydrogel pieces were completely separated was measured at a crosshead speed of 10 mm/minute using a 50 N load cell.

Referring to Fig. 8, the smaller the particle size of the PHA particles, the higher the adhesive strength. In particular, when the particle size of the PHA particles was 300 nm or less and the concentration of the PHA particles was 3 to 10% (w/v), significantly high adhesive strength was exhibited.

These results also support that it is necessary to adjust the particle size of PHA particles to increase adhesive strength to biological tissue and that the medical composition (tissue adhesion composition) according to the present disclosure exhibits strong adhesive strength even in a moist environment.

### Test Example 6: Evaluation of wound recovery 1

The PHA particles with a particle size of 178 nm obtained in Example 1 ([P(3HB-co-4HB)], 4HB repeat unit: 17% by weight, molecular weight: 687,000 g/mole) were dissolved (dispersed) in a 0.1 M PBS buffer (pH 7.4) at a concentration of 7.8% (w/v) to obtain a composition, which was subjected to a wound recovery test. The results are shown in Figs. 9 to 11. Specifically, Sprague Dawley (SD) rats within 7 weeks of age or younger, matched for gender and age, and sterilized surgical instruments were prepared. In addition, the composition in which the PHA particles were dissolved (dispersed) at a concentration of 7.8% (w/v) was sterilized with ultraviolet rays. Next, the prepared rats were anesthetized using a respiratory anesthesia machine filled with an Ifran solution. The back of each anesthetized rat was shaved with a razor, a circular wound area was created using a biopsy punch (Kai Medical, Japan) with a diameter of 8 mm, the UV-sterilized composition was applied, and a sterilized waterproof film was attached to prevent scratching. Thereafter, the wound recovery process was filmed at the same time for 21 days using a camera, and the wound area was calculated using the Image J program. In addition, on the 7^{th} and 14^{th} days from the initiation of the test, they were euthanized using carbon dioxide. The skin tissue around the wound site was cut into a size of 10 mm × 10 mm and fixed in a formalin solution, followed by hematoxylin and eosin (H&E) staining and Masson's trichrome (MT) staining. Images of the stained area were observed using a Leica microscope. In such an event, no treatment (NT) was used as a negative control group, and fibrin glue (Green Cross, Korea) (FG) was used as a positive control group.

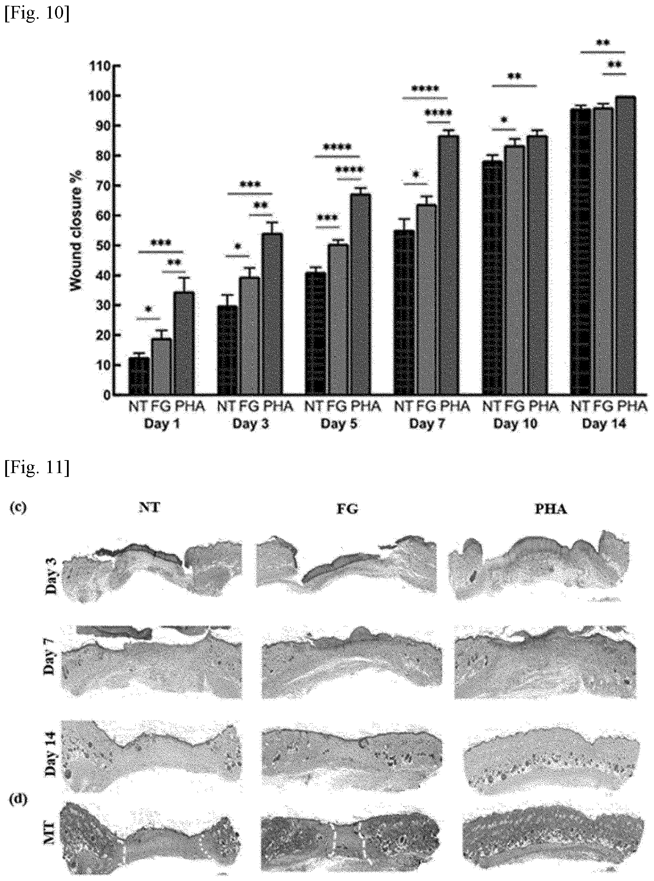

Referring to Figs. 9 and 10, the medical composition (PHA) according to the present disclosure reduced the wound site in a faster time than the control groups.

Referring to Fig. 11 (c) (hematoxylin and eosin (H&E) staining result), when treated with the medical composition (PHA) according to the present disclosure, it was observed that wound healing proceeded quickly, and numerous new blood vessels were created. These blood vessels newly created support the creation of temporary granulation tissue, which can deliver nutrients and oxygen to the expanded tissue needed to support the healing process while enhancing the growth of new tissue. In addition, the angiogenic process can help remove waste and dead cells from the wound site, speeding up the healing process. Meanwhile, when treated with the medical composition (PHA) according to the present disclosure, faster re-epithelialization (epidermal restoration) took place as compared with the control groups. In particular, the epithelial tongue newly created from the third day could be observed only in the group treated with the medical composition (PHA) according to the present disclosure.

Referring to Fig. 11 (d) (Masson's trichrome (MT) staining result), when treated with the medical composition (PHA) according to the present disclosure, the granulation tissue was remodeled more quickly into a more mature collagen content structure.

These results support that the medical composition (wound healing composition) according to the present disclosure has wound healing efficacy.

### Test Example 7: Evaluation of wound recovery 2

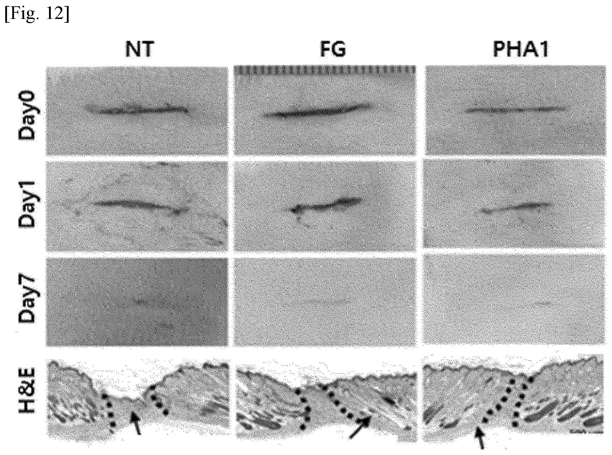

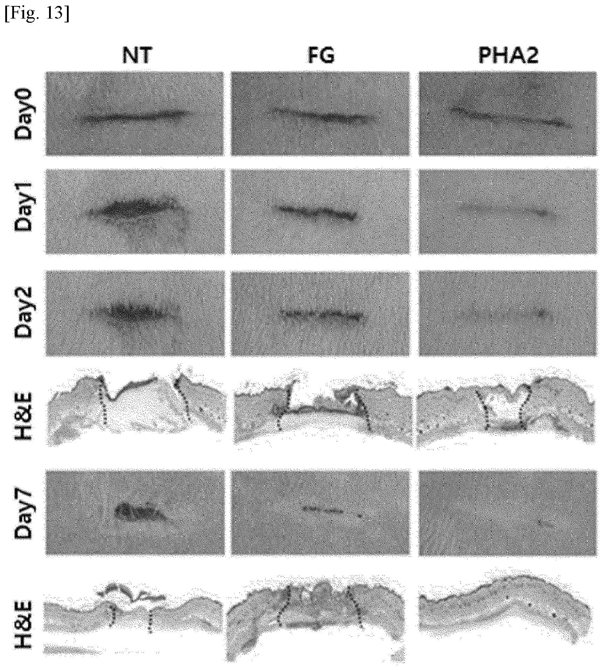

The PHA particles with a particle size of 178 nm obtained in Example 1 ([P(3HB-co-4HB)], 4HB repeat unit: 17% by weight, molecular weight: 687,000 g/mole) and the PHA particles with a particle size of 180 nm ([P(3HB-co-4HB)], 4HB repeat unit: 8.7% by weight, molecular weight: 390,000 g/mole) obtained in Example 3 were dissolved (dispersed) in a 0.1 M PBS buffer (pH 7.4) at a concentration of 25% (w/v) to obtain compositions (PHA1 (4HB repeat unit: 17% by weight) and PHA2 (4HB repeat unit: 8.7% by weight)), respectively, which were each subjected to a wound recovery test. The results are shown in Figs. 12 and 13. Specifically, Sprague Dawley (SD) rats were anesthetized with isoflurane, their backs were shaved, and the skin was incised in a size of 1.5 cm in length and 3.0 mm in depth to create a narrow skin wound. Next, 10 µl of each of the PHA 1 composition and PHA2 composition were applied to the wound site, and the wound site was fixed for 3 minutes. After 7 days, the rats were euthanized, and the skin was incised in a size of 1 cm × 2 cm to encompass the wound site. Subsequently, the incised skin was fixed in a p-formaldehyde solution (3.7% by weight) and embedded in paraffin, and the skin cross-section was stained with hematoxylin and eosin (H&E). Thereafter, images of the stained area were observed using a Leica microscope.

Referring to Figs. 12 and 13, the medical compositions (PHA1 and PHA2) according to the present disclosure allowed the wound site to recover cleanly as compared with the control groups.

### Test Example 8: Evaluation of wound recovery 3 (analysis of differentially expressed genes (DEGs) of PHA particles in in vitro cell lines)

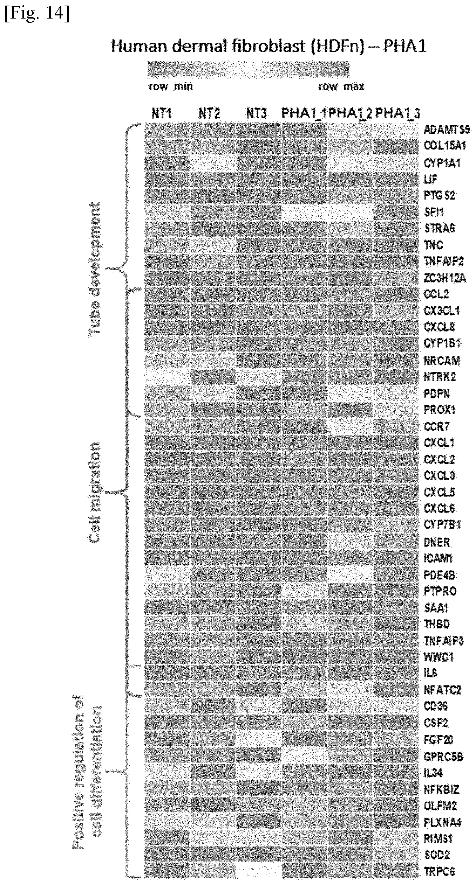

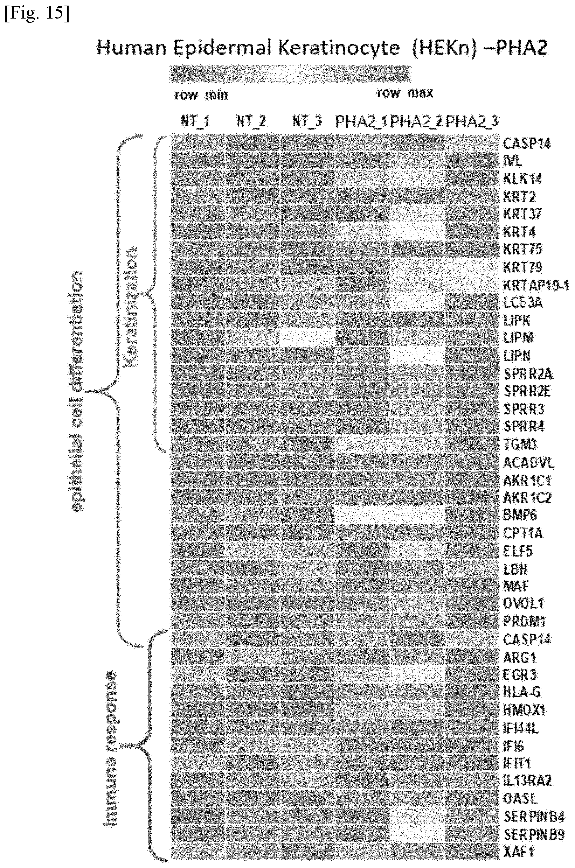

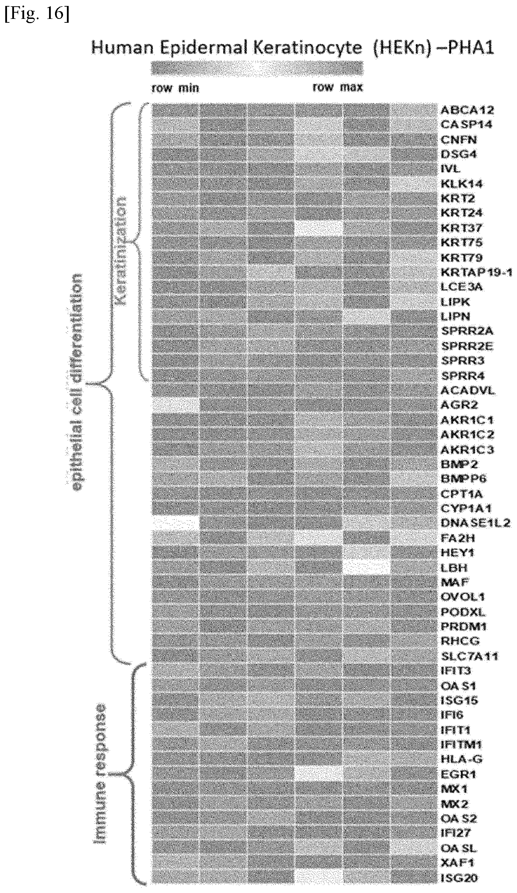

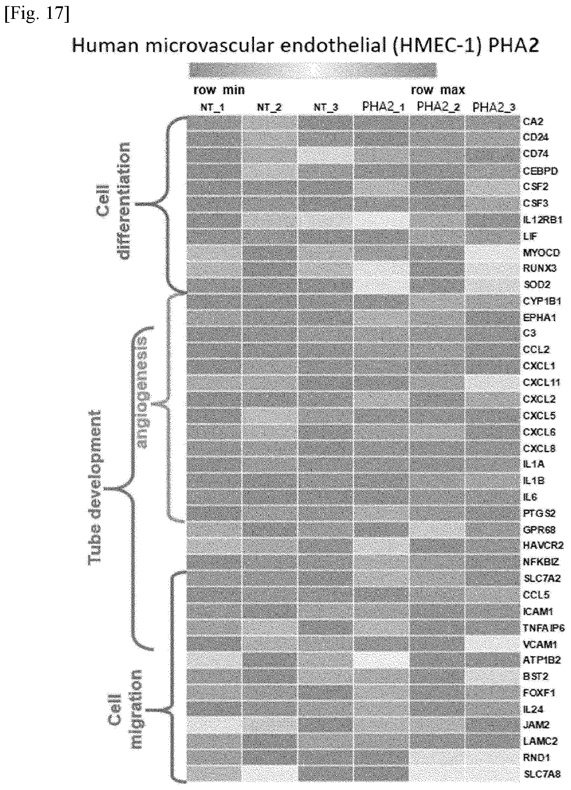

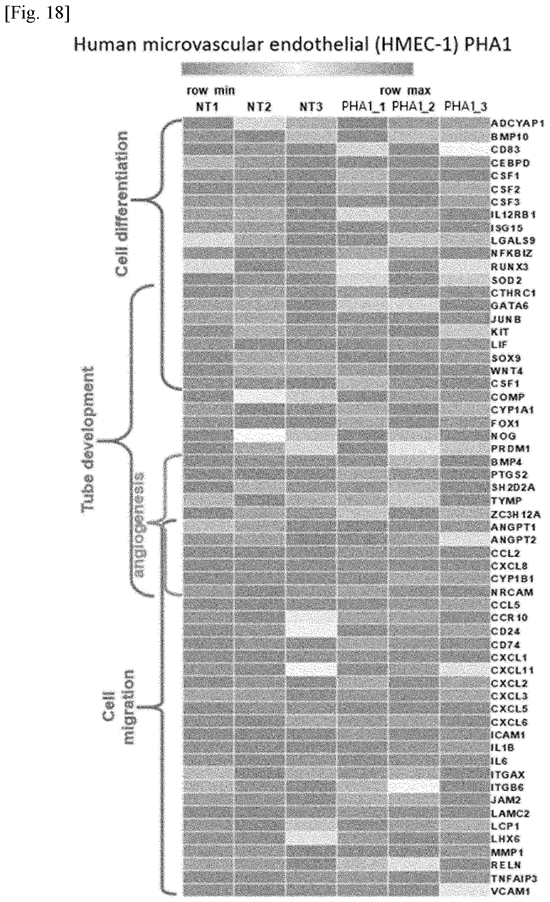

The PHA particles with a particle size of 178 nm obtained in Example 1 ([P(3HB-co-4HB)], 4HB repeat unit: 17% by weight, molecular weight: 687,000 g/mole) and the PHA particles with a particle size of 180 nm ([P(3HB-co-4HB)], 4HB repeat unit: 8.7% by weight, molecular weight: 390,000 g/mole) obtained in Example 3 were dissolved (dispersed) in a 0.1 M PBS buffer (pH 7.4) at a concentration of 25% (w/v) to obtain compositions (PHA1 (4HB repeat unit: 17% by weight) and PHA2 (4HB repeat unit: 8.7% by weight)), respectively. DEGs analysis was performed on these compositions to determine the genes involved in human wound healing that are affected by PHA particles. The results are shown in Figs. 14 to 18. Specifically, HMEC01, a human vascular cell line, and HDfn, a human skin fibroblast cell, were cultured in PRMI 1640 containing 10.0% by volume of FBS and 1% by volume of penicillin-streptomycin (under conditions with LC nanoclusters in a humidified incubator at 37°C and 5% CO₂). In addition, 10 ml of a cell suspension with a density of 1.5×10⁵ cells/ml was added to each round cell culture dish having a diameter of 10 cm, and the outer area was filled with the cell culture medium and incubated at 37°C and 5% CO₂ for 24 hours. After incubation, each of the PHA1 composition and PHA2 composition was added to the cell culture medium and further cultured at 37°C and 5% CO₂ for 24 hours. After further incubation, the medium was discarded, and 1 ml of trizol was added, which was rapidly frozen at -70°C, and a DEGs test analysis was requested to ROKIT Genomics Co., Ltd. to obtain the results.

Referring to Figs. 14 to 18, genes mainly related to angiogenesis and wound healing were overexpressed in the groups treated with the PHA particles.

### Test Example 9: Evaluation of hemostasis efficacy 1

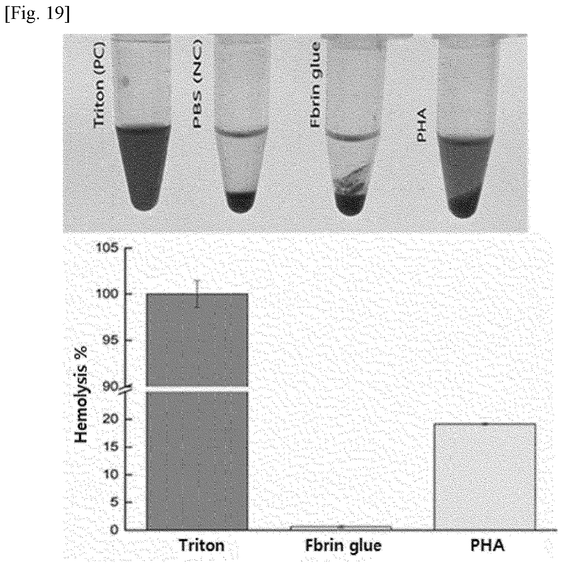

The PHA particles with a particle size of 178 nm obtained in Example 1 ([P(3HB-co-4HB)], 4HB repeat unit: 17% by weight, molecular weight: 687,000 g/mole) were dissolved (dispersed) in a 0.1 M PBS buffer (pH 7.4) at a concentration of 7.8% (w/v) to obtain a composition, which was subjected to a hemostasis performance test. The results are shown in Fig. 19. Specifically, rabbit whole blood containing a sodium citrate solution (3.8%) was centrifuged to separate red blood cells (RBCs) and platelet-rich plasma (PRP) for hemolysis analysis and plasma coagulation analysis, respectively. Next, the red blood cells (RBCs) were washed four times with a saline solution, and the red blood cells diluted to 500 µl (RBCs of 1 ml and 9 ml of a saline solution) were added to a microtube containing 100 mg of the composition (PHA composition). In such an event, 100 µl of 0.1% Triton and 100 µl of PBS were each used as a positive control (PC) group and a negative control (NC) group. 100 µl of fibrin glue was used as a reference group. The microtube was incubated at 37°C for 1 hour and then centrifuged at 3,500 rpm for 10 minutes. Thereafter, the supernatant thus obtained was analyzed for optical density (O.D.) using a microplate reader at 540 nm. Hemolysis (%) was calculated according to the following Equation 1 (N = 3). Hemolysis (%) = {(O.D. sample - O.D. negative control group)/(O.D. positive control group - O.D. negative control group)} × 100

Referring to Fig. 19, the medical composition (hemostasis composition) according to the present disclosure had excellent hemostatic efficacy.

### Test Example 10: Evaluation of hemostasis efficacy 2

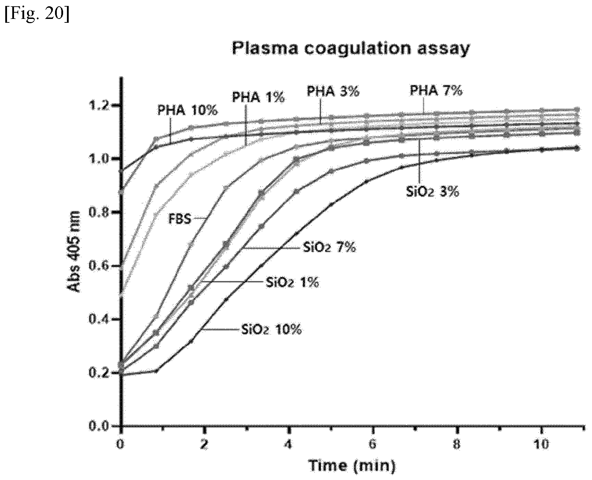

The PHA particles with a particle size of 178 nm obtained in Example 1 ([P(3HB-co-4HB)], 4HB repeat unit: 17% by weight, molecular weight: 687,000 g/mole) were dissolved (dispersed) in a 0.1 M PBS buffer (pH 7.4) at a concentration of 1% (w/v), 3% (w/v), 7% (w/v), and 10% (w/v) to obtain compositions, respectively, which were each subjected to a hemostasis performance test. The results are shown in Fig. 20. Specifically, rabbit whole blood containing a sodium citrate solution (3.8%) was centrifuged to separate red blood cells (RBCs) and platelet-rich plasma (PRP) for hemolysis analysis and plasma coagulation analysis, respectively. Next, 180 µl of plasma was added to the microtube into which each of the compositions prepared at the respective concentrations (PHA compositions) and 20 µl of nanoparticle suspension silica had been added. Thereafter, the microtube was incubated at 37°C for 30 minutes and then centrifuged at 6,000 rpm for 5 minutes. Next, 100 µl of each supernatant was transferred to a well of a 96-well flat bottom plate and recalcified with 65 µl of a 50 mM CaCl₂ solution. Subsequently, the degree of fibrin polymerization was evaluated by measuring absorbance at 405 nm every 50 seconds for 1 hour using a plate reader.

Referring to Fig. 20, the medical composition (hemostasis composition) according to the present disclosure had a high degree of fibrin polymerization, indicating excellent hemostatic efficacy.

### Test Example 11: Evaluation of inhibition of bacterial infection efficacy

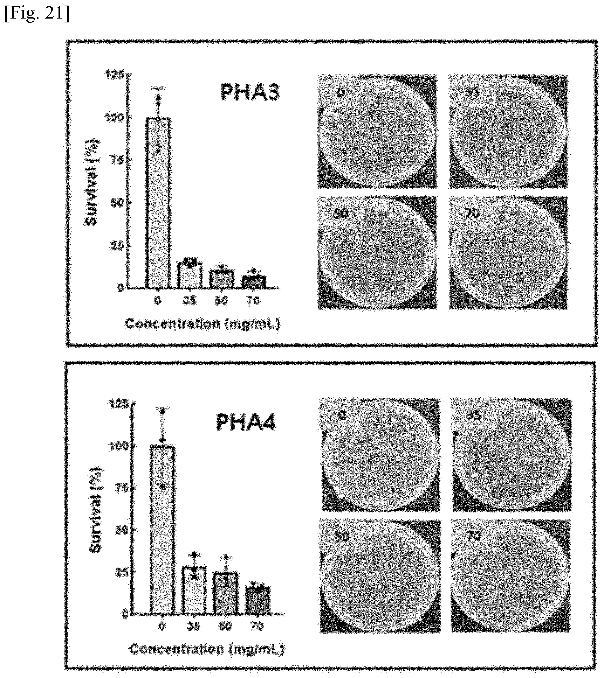

The PHA particles with a particle size of 178 nm obtained in Example 1 ([P(3HB-co-4HB)], 4HB repeat unit: 17% by weight, molecular weight: 687,000 g/mole) and the PHA particles with a particle size of 180 nm ([P(3HB-co-4HB)], 4HB repeat unit: 8.7% by weight, molecular weight: 390,000 g/mole) obtained in Example 3 were dissolved (dispersed) in a 0.1 M PBS buffer (pH 7.4) at a concentration of 30% (w/v), 50% (w/v), and 70% (w/v) to obtain compositions (PHA3 (4HB repeat unit: 17% by weight) and PHA4 (4HB repeat unit: 8.7% by weight)), respectively, which were each evaluated for antibacterial activity. The results are shown in Fig. 21. Specifically, when the OD 600 value reached 0.5, each PHA composition was added to 0.2 ml of an *E. coli* solution, which was then applied to an LB solid medium. Subsequently, after it was incubated at 37°C for 12 hours, the degree of colony formation for *E. coli* was analyzed by determining the number of colonies formed.

Referring to Fig. 21, the number of colonies of the medical compositions (PHA3 and PHA4) according to the present disclosure decreased as the PHA concentration increased. These results support that the medical composition (bacterial infection inhibition composition) according to the present disclosure had inhibition of bacterial infection efficacy.

## Claims

1. A medical composition, which comprises a polyhydroxyalkanoate (PHA) having a particle size of 10,000 nm or less and comprising a repeat unit derived from 3-hydroxybutyrate (3HB) in an amount of 40% by weight or more based on the total weight.

2. The medical composition of claim 1, which has a tissue adhesive strength of 15 kPa or more when measured at a relative humidity of 100% and room temperature.

3. The medical composition of claim 1, wherein the polyhydroxyalkanoate (PHA) further comprises a repeat unit derived from 4-hydroxybutyrate (4HB).

4. The medical composition of claim 3, wherein the content of the repeat unit derived from 4-hydroxybutyrate (4HB) is 0.1 to 60% by weight based on the total weight of the polyhydroxyalkanoate (PHA).

5. The medical composition of claim 1, wherein the polyhydroxyalkanoate (PHA) is a poly(3-hydroxybutyrate-co-4-hydroxybutyrate) copolymer.

6. The medical composition of claim 1, wherein the polyhydroxyalkanoate (PHA) has a molecular weight of 10,000 to 1,200,000 g/mole.

7. The medical composition of claim 1, wherein the concentration of the polyhydroxyalkanoate (PHA) is 0.1 to 75% (w/v).

8. The medical composition of claim 1, which is used for hemostasis.

9. The medical composition of claim 1, which is used for wound healing.

10. The medical composition of claim 1, which is used for tissue adhesion.

11. The medical composition of claim 1, which is used for inhibition of bacterial infection.

12. A process for preparing a medical composition, which comprises:
dissolving a polyhydroxyalkanoate (PHA) in a solvent to prepare a dispersed phase solution;
preparing a continuous phase solution comprising a surfactant;
passing the dispersed phase solution through a membrane having a pore size of 10,000 nm or less to form an emulsion in which dispersed phase particles are dispersed in the continuous phase solution; and
solidifying the emulsion to obtain polyhydroxyalkanoate (PHA) particles with a particle size of 10,000 nm or less.

13. The process for preparing a medical composition of claim 12, wherein the content of the polyhydroxyalkanoate (PHA) contained in the dispersed phase solution is 0.01 to 5% by weight based on the total weight of the dispersed phase solution.

14. The process for preparing a medical composition of claim 12, wherein the dispersed phase solution passes through the membrane at a pressure of 2.5 to 320 kPa.

15. A process for preparing a medical composition, which comprises:
dissolving a polyhydroxyalkanoate (PHA) in a solvent to prepare a dispersed phase solution;
preparing a continuous phase solution comprising a surfactant;
mixing the dispersed phase solution and the continuous phase solution to form a premix emulsion;
injecting the premix emulsion into a high-pressure dispersion device (microfluidizer) to form an emulsion in which polyhydroxyalkanoate (PHA) particles with a particle size of 10,000 nm or less are dispersed; and
solidifying the emulsion to obtain the polyhydroxyalkanoate (PHA) particles.

16. The process for preparing a medical composition of claim 15, wherein the pressure applied to the premix emulsion by the high-pressure dispersion device is 1 to 30 kpsi.
